# EUROPEAN PATENT APPLICATION

(11) **EP 3 875 602 A1**
(43) Date of publication of application: **08.09.2021**
(21) Application number: 20161291.8
(22) Date of filing: 05.03.2020
(51) Int. Cl.: C12Q 1/6804, C12Q 1/6816

(54) **NUCLEIC ACID FLUORESCENCE DETECTION**

(71) Applicant: Nederlandse Organisatie voor toegepast- natuurwetenschappelijk Onderzoek TNO, 2595 DA 's-Gravenhage (NL)
(72) Inventor: Van Leeuwen, Hans Christiaan, 2595 DA s'-Gravenhage (NL)
(74) Representative: V.O.

(57) **Abstract**

The invention relates to a nucleic acid detection system, a diagnostic device, use of the nucleic acid detection system as a diagnostic agent, a kit-of-parts for detecting nucleic acids, a method for detecting nucleic acids, and a method for diagnosing a disease state of a subject. The nucleic acid detection system comprises a CRISPR-Cas system which comprises an effector protein and one or more guide RNAs having a guide sequence, the guide sequence being capable of targeting the effector protein to a target sequence of a target, and the effector protein exhibiting target-activated nucleic acid cleavage activity capable of cleaving nucleic acid reporter molecules to generate nucleic acid fragments; and a polymerase exhibiting catalytic activity capable of transferring nucleotides to the fragments to form polynucleotide tails having metal-binding sites capable of chelating metal, wherein preferably the detection system is a nucleic acid fluorescence detection system.

## Description

The invention relates to a nucleic acid detection system, a diagnostic device, use of the nucleic acid detection system as a diagnostic agent, a kit-of-parts for detecting nucleic acids, a method for detecting nucleic acids, and a method for diagnosing a disease state of a subject.

Clustered Regularly Interspaced Short Palindromic Repeats (CRISPR) and CRISPR-associated (CRISPR-Cas) nucleases constitute a class of adaptive immune systems used by bacteria and archaea. A number of CRISPR-Cas systems have been adapted to, for example, genome engineering, serving as programmable nucleases cleaving desired genomic sequences or as specific DNA binders enabling transcriptional regulation, base editing, or imaging.

In addition, CRISPR-Cas systems can be used in detecting a myriad of pathogens, such as viral and bacterial pathogens, by harnessing cleaved nucleic acid fragments for use in diagnostic testing.

Several diagnostic CRISPR-Cas assays exist, such as DETECTR (DNA Endonuclease Targeted CRISPR Trans Reporter), SHERLOCK (Specific High Sensitive Enzymatic Reporter) and HOLMES (one-HOur Low-cost Multipurpose highly Efficient System). These assays rely on synthetic RNA/DNA oligonucleotides that have a reporter fluorophore (donor) on one end and a quencher (acceptor) in proximity on the other end. Intact, these fluorescence resonance energy transfer (FRET) based reporters show no fluorescence, but upon DNA or RNA cleavage by an activated Cas effector protein, the released donor fluorophore is no longer quenched, and a fluorescent signal is measurable. Ideally, in the FRET pair there should be extensive overlap between the donor emission-spectrum and the acceptor absorption-spectrum, but no overlap in the excitation spectra. Isolating these fluorescent signals requires a narrow set of filters in a fluorometer which discards portions of the spectra thereby affecting the sensitivity. Moreover, measuring FRET fluorescence directly in biological samples adds interference sources causing absorbance, autofluorescence or light scattering.

For example, WO 2019/104058 relates to a method for detecting target DNA by using labeled single-stranded detector DNA comprising a FRET pair, or fluorescence-emitting dye pair. The method requires a class 2 type V CRISPR-Cas effector protein (Cas12).

WO 2019/071051, WO 2019/126577, WO 2019/148206, and WO 2018/170340, relate to nucleic acid detection systems and methods using the same for detecting nucleic acids that are diagnostic for a disease state. The nucleic acid detection systems comprise an oligonucleotide which may comprise a detection agent, such as colloidal gold, that changes color when the agent moves from aggregated condition to dispersed condition in solution. It would be desirable to design a nucleic acid detection system which does not require an expensive metal.

CN 106282323 is directed to a DNA fluorescence analysis method that uses polythymine-templated copper nanoparticles comprising target DNA. The particles are prepared by using magnetic beads which provide for a template for said particles. The method requires *inter alia* the use of a magnetic separator as well as multiple repetitive washing steps. It would be desirable to design the method such that it becomes portable and easy to use by first responders.

DNA fluorescence analysis methods known from the art, such as diagnostic CRISPR-Cas assays that are designed to use FRET, may require the use of expensive measuring equipment, such as fluorometers, and/or expensive metals. Such methods are particularly inconvenient for use by first responders as a quick initial method for detecting pathogens.

It is an objective of the invention to provide a nucleic acid detection system which is easy to use by first responders for detecting nucleic acids, in particular pathogenic nucleic acids, near a patient or on site.

A further objective of the invention is to provide a method for detecting nucleic acids which is easy to perform and does not require high-end equipment.

The inventors found that one or more of these objectives can, at least in part, be met by combining CRISPR technology and photoluminescence, in particular fluorescence. Fluorescent (nano)clusters exhibit great potential for fluorescent bioassays thanks to the advantages of high fluorescence yield, good photo-stability and a visible emission (orange/red) when excited with ultraviolet light (Luo, *et al.* 2017). Accordingly, a versatile, rapid and portable system for the detection of nucleic acids is disclosed herein, in particular a fluorescence detection system.

Accordingly, in a first aspect of the invention there is provided a nucleic acid detection system, comprising: a CRISPR-Cas system which comprises an effector protein and one or more guide RNAs having a guide sequence, the guide sequence being capable of targeting the effector protein to a target sequence of a target, and the effector protein exhibiting target-activated nucleic acid cleavage activity capable of cleaving nucleic acid reporter molecules to generate nucleic acid fragments; and a polymerase exhibiting catalytic activity capable of transferring nucleotides to the fragments to form polynucleotide tails having metal-binding sites capable of chelating metal, wherein preferably the detection system is a nucleic acid fluorescence detection system.

In a further aspect of the invention, there is provided a use of a nucleic acid detection system as described herein as a diagnostic agent.

In yet a further aspect of the invention, there is provided a diagnostic device, comprising one or more nucleic acid detection systems as described herein, and optionally comprising a source of electromagnetic radiation.

In yet a further aspect of the invention, there is provided a kit-of-parts for detecting nucleic acids, comprising:
i) a first container (A) which comprises:
   a CRISPR-Cas system, preferably as described herein, and preferably further comprising nucleic acid reporter molecules, preferably as described herein, preferably single-stranded nucleic acids;
ii) a second container (B) which comprises:
   polymerase and nucleotides, preferably as described herein;
iii) a third container (C) which comprises:
   metal, preferably as described herein, preferably together with a reductant, and
v) optionally a fourth container (D) which comprises:
   a source of electromagnetic radiation.

In yet a further aspect of the invention, there is provided a method for detecting nucleic acids, preferably pathogenic DNA, comprising:
i) target-activating the nucleic acid cleavage activity of a CRISPR-Cas system as described herein and allowing the activated CRISPR-Cas system to generate nucleic acid fragments by cleaving nucleic acid reporter molecules, preferably as described herein;
ii) adding polymerase and nucleotides to at least part of the nucleic acid fragments to form a polynucleotide tail attached to the nucleic acid fragments, wherein preferably the polymerase and/or nucleotides are as described herein;
iii) adding metal preferably as described herein, preferably together with a reductant to bind the metal to the polynucleotide tail, thereby forming metal clusters, and
iv) exposing the metal clusters to electromagnetic radiation, preferably ultraviolet radiation, in particular having a wavelength of 300-400 nm.

In yet a further aspect of the invention, there is provided a method for detecting and/or monitoring nucleic acids in a subject, preferably *ex vivo* or *in vitro,* wherein preferably the nucleic acids are microbial nucleic acids, more preferably pathogenic nucleic acids, comprising the steps of the method for detecting nucleic acids as described herein.

In yet a further aspect of the invention, there is provided a method for diagnosing a disease state of a subject, preferably *ex vivo* or *in vitro,* comprising the steps of the method for detecting nucleic acids as described herein.

The terms "to bind" and "binding" as used herein (*e.g.,* binding an effector protein to a target nucleic acid) are meant to refer to a non-covalent interaction between macromolecules (*e.g.,* between a protein and a nucleic acid, and between a guide RNA and a target nucleic acid). While in a state of non-covalent interaction, the macromolecules are "associated", "interacting" or "binding" (*e.g.,* when a molecule is said to interact with another molecule, it is meant that the both molecules bind in a non-covalent manner). Not all components of a binding interaction need be sequence-specific (*e.g.,* contacts with phosphate residues in a DNA backbone), but some portions of a binding interaction may be sequence-specific.

The term "chelating" are used herein (*e.g.,* metal-binding sites capable of chelating metal) is meant to refer to one or more coordinate bonds.

The term "guide sequence" as used herein is meant to refer to a nucleotide sequence of a guide RNA molecule. The guide sequence is considered to be the proverbial key to bind an effector protein, comprising the guide RNA, to a target molecule, *e.g.,* a nucleic acid, through sequence pairing.

The term "nucleic acid" as used herein is meant to refer to a polymeric form of nucleotides of any length, *e.g.,* ribonucleotides and deoxyribonucleotides. The term is used to include microbial nucleic acids, such as pathogenic nucleic acids. The term encompasses single-stranded DNA, double-stranded DNA, multi-stranded DNA, single-stranded RNA, double-stranded RNA, multi-stranded RNA, genomic DNA, cDNA, DNA-RNA hybrids, and polymers comprising purine and pyrimidine bases or other natural, chemically or biochemically modified, non-natural, or derivatized nucleotide bases.

The term "protein" as used herein is meant to refer to a polymeric form of amino acids of any length, which can include coded and non-coded amino acids, chemically or biochemically modified or derivatized amino acids, and proteins having modified backbones.

The term "target sequence" as used herein is meant to refer to a nucleotide sequence of a target (*i.e.,* a target molecule) to which a guide RNA having a sequence with a certain degree of complementarity with the target sequence, may bind an effector protein to the target (molecule).

In accordance with the invention, an innovative nucleic acid detection system is provided, in particular comprising CRISPR technology, for detecting, for example, pathogenic DNA, having good handleability and which is easy to use.

The invention provides a nucleic acid detection system, comprising a CRISPR-Cas system which comprises an effector protein and one or more guide RNAs having a guide sequence, the guide sequence being capable of targeting the effector protein to a target sequence of a target, and the effector protein exhibiting target-activated nucleic acid cleavage activity capable of cleaving nucleic acid reporter molecules to generate nucleic acid fragments; and a polymerase exhibiting catalytic activity capable of transferring nucleotides to the fragments to form polynucleotide tails having metal-binding sites capable of chelating metal.

The nucleic acid detection system as described herein may be a nucleic acid fluorescence detection system.

The nucleic acid detection system of the invention comprises a CRISPR-Cas system. In general, a CRISPR-Cas system is characterized by components that promote the formation of a so-called CRISPR complex, albeit at the site of a target sequence. Typically, a CRISPR complex comprises a complex of effector protein and guide RNA. As used herein, the CRISPR-Cas system comprises an effector protein and one or more guide RNAs that are capable of guiding the effector protein to bind to a target molecule, such as a nucleic acid. That is, the guide RNA has a guide sequence which is capable of targeting an effector protein to a target sequence of a target. Typically a guide-target hybrid is formed. In particular, the CRISPR-Cas system comprises guide RNA having a guide sequence which is capable of targeting an effector protein to a target sequence, wherein preferably the target sequence is a (specific) nucleotide sequence of a target molecule, such as a nucleotide sequence of a nucleic acid, *e.g.,* a pathogenic nucleic acid.

CRISPR-Cas systems are commonly categorized in two classes (1 and 2). Types and subsequent subtypes constitute either class. The categorization of CRISPR-Cas systems is based on, for example, the constitution of effector modules of single, large, multidomain proteins that are generally derived from genetic elements. Whereas class 1 CRISPR-Cas systems contain a multi-subunit Cas nuclease, or effector protein, class 2 CRISPR-Cas systems are characterized by a single-subunit effector protein. Exemplary effector proteins that belong to class 2 CRISPR-Cas systems are type II Cas9 and Cas9-like proteins, type V Cas12 and Cas12-like proteins, such as subtype V-A Cas12 (Cpf1, or Cas12a), subtype V-B Cas12 (Cas12b, or C2c1) and subtype V-C Cas12 (C2c3), and type VI Cas13 and Cas13-like proteins, such as Cas 13a (C2c2) and Cas13b (C2c6).

In particular, the detection system as described herein comprises a class 2 CRISPR-Cas system. Preferably, the CRISPR-Cas system is a type V or VI system. More preferably, the CRISPR-Cas system is a class 2 type V system. In case of a class 2 system, the effector protein may particularly be Cas12 or an effector protein having similar cleavage activity as Cas12. The Cas12 or Cas12-like effector protein may, for example, be selected from Cas12a, Cas12b, Cas12c, Cas12d and Cas12e. Preferably, the CRISPR-Cas system comprises a C12a effector protein or an effector protein having similar cleavage activity as Cas12a.

The nucleic acid detection system as described herein may comprise more than one CRISPR-Cas system, for example, wherein the more than one CRISPR-Cas systems are different. Preferably, the detection system comprises one CRISPR-Cas system.

The programmability and specificity of the RNA-guided class 2 Cas effector proteins, such as Cpf1, make them suitable switchable nucleases for specific cleavage of nucleic acids. The class 2 Cas effector proteins, such as Cpf1, may be engineered to provide and take advantage of improved collateral non-specific cleavage of DNA, preferably ssDNA. Accordingly, engineered class 2 Cas effector proteins, such as Cpf1, may provide suitable platforms for nucleic acid detection.

The CRISPR-Cas system comprising an Cpf1 effector protein may be from an organism from a genus comprising *Streptococcus, Campylobacter, Nitratifractor, Staphylococcus, Parvibaculum, Roseburia, Neisseria, Gluconacetobacter, Azospirillum, Sphaerochaeta, Lactobacillus, Eubacterium, Corynebacter, Carnobacterium, Rhodobacter, Listeria, Paludibacter, Clostridium, Lachnospiraceae, Clostridiaridium, Leptotrichia, Francisella, Legionella, Alicyclobacillus, Methanomethyophilus, Porphyromonas, Prevotella, Bacteroidetes, Helcococcus, Letospira, Desulfovibrio, Desulfonatronum, Opitutaceae, Tuberibacillus, Bacillus, Brevibacilus, Methylobacterium* or *Acidaminococcus.*

The effector protein as used herein may be a chimeric effector protein comprising a first fragment from a first effector protein (*e.g.,* a Cas12a) and a second fragment from a second effector protein (*e.g.,* a Cas 12b), and wherein the first and second effector proteins are different. At least one of the first and second fragments of the chimeric effector protein may be from an effector protein from an organism from a genus comprising any of the herein mentioned organisms.

The CRISPR-Cas system in the detection system as described herein comprises one or more guide RNAs (gRNAs) which are capable of guiding the effector protein to bind to specific target molecules, such as target pathogenic nucleic acids.

Guide RNA as used herein is characterized by comprising any nucleotide sequence, or guide sequence, having sufficient complementarity with the nucleotide sequence of a target molecule to hybridize (and pair), and to direct sequence-specific binding of a molecule-targeting effector protein to the target molecule. The guide sequence may be a full-length guide sequence or a truncated guide sequence.

The degree of complementarity of the guide sequence to a given target sequence, when optimally aligned using a suitable alignment algorithm, is 50 % or more, 60 % or more, 75 % or more, 80 % or more, 85 % or more, 90 % or more, 95 % or more, 97.5 % or more, or 99 % or more. In particular, the degree of complementarity may be 75 % or more, such as 85-100 %, 90-100 %, or 95-100 %. Preferably, the degree of complementarity is 97.5 % or more, 98 % or more, 98.5 % or more, 99 % or more, 99.5 % or more, or 99.7 % or more. More preferably, the degree of complementarity is about 100 %. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (*e.g.,* the Burrows Wheeler Aligner), ClustalW, Clustal X, BLAT, Novoalign (Novocraft Technologies; available at www.novocraft.com), ELAND (Illumina, San Diego, CA), SOAP (available at soap.genomics.org.cn), and Maq (available at maq.sourceforge.net).

The CRISPR-Cas system in the detection system as described herein may comprise one or more gRNAs which are capable of forming a wobble base pair with a target sequence. That is, such gRNAs do not completely follow Watson-Crick base pairing. The CRISPR-Cas system may comprise one or more gRNAs that do follow Watson-Crick base pairing and one or more gRNAs that do not.

The ability of a guide sequence to direct sequence-specific binding of, for example, a nucleic acid-targeting effector protein to a target nucleic acid sequence, may be assessed by any suitable assay. For example, the components of a nucleic acid-targeting CRISPR system sufficient to form a nucleic acid-targeting complex, including the guide sequence to be tested, may be provided to a host cell having the corresponding target sequence, such as by transfection with vectors encoding the components of the nucleic acid-targeting complex, followed by an assessment of preferential targeting (*e.g.,* cleavage) within the target nucleic acid sequence. Similarly, cleavage of a target sequence (or a sequence in the vicinity of the target sequence) may, for example, be evaluated in a test tube by providing the target sequence, components of a nucleic acid-targeting complex, including the guide sequence to be tested and a control guide sequence different from the test guide sequence, and comparing binding or rate of cleavage at or in the vicinity of the target sequence between the test and control guide sequence reactions. Other assays are possible, and will occur to those skilled in the art. A guide sequence, and hence a nucleic acid-targeting guide RNA may be selected to target any target sequence, for example, any target sequence of target nucleic acids.

The total length of guide RNA may be at least 10 nucleotides and may be 100 nucleotides or less, such as 15-85 nucleotides. In particular, the guide RNA is at least 15 nucleotides, such as 20-70, such as 30-65, or 40-50. Preferably, the length of guide RNA is 35 or more and 45 or less.

The length of the guide sequence of the guide RNA may be at least 10 nucleotides and may be 100 nucleotides or less, such as 15-50 nucleotides. In particular, the guide sequence is at least 15 nucleotides, such as 16-35, such as 17-30. Preferably, the guide sequence is 18 or more and 25 or less.

The guide RNA may comprise naturally and/or non-naturally occurring nucleotides, nucleotide analogs, and/or chemical modifications. The non-naturally occurring nucleotides, nucleotide analogs, and/or chemical modifications may be located outside the guide sequence. Non-naturally occurring nucleotides and/or nucleotide analogs may be modified at the ribose, any or all of the phosphate groups, and/or nitrogenous base.

The guide RNA may comprise one or more hairpin loop motifs and/or one or more stem-loops, depending on the guide RNA and/or effector protein used.

The guide RNA, such as crRNA, may comprise, consist essentially of, or consist of repetitive sequences and one or more a guide sequences. The repetitive sequence may be an inverted repeat (IR) sequence or a direct repeat (DR) sequence. Either IR or DR sequence may be located upstream (*i.e.,* 5' terminus) from the guide sequence, or downstream (*i.e.,* 3' terminus) from a guide sequence.

The target sequence may be located in the nucleus, organelles or cytoplasm of a cell, and may include nucleic acids in or from mitochondrial, organelles, vesicles, liposomes or particles present within the cell. The target sequence does not have to be located intracellular. For example, the target sequence may be extracellular, such as in spores. In case the target sequence is a virus sequence, it may be located in a protein structure, such as a viral capsid.

The CRISPR-Cas system of the detection system as described herein may comprise the following gRNA(s): 5'-UAA UUU CUA CUA AGU GUA GAU CAU AUU AUA UCG AGC CAC AGC-OH-3' (crRNA1), and/or 5'-UAA UUU CUA CUA AGU GUA GAU UGC ACC GGA AGC UUU UAA UUA C-OH-3' (crRNA2), and/or 5'-UAA UUU CUA CUA AGU GUA GAU GCU CAA UAG GAA UCU GCA GC-OH-3' (crRNA3). These gRNAs specifically target *Bacillus anthracis.*

A protospacer adjacent motif (or, "PAM") or any PAM-like motif directs binding of the effector protein to the target sequence of interest. The PAM may be a 5' PAM (*i.e.,* located upstream of a 5' end of a target molecule) or a 3' PAM (*i.e.,* located downstream of a 5' end of a target molecule). Recognition of a 3' PAM or 5' PAM depends on the effector protein. For example, some Cpf1 effector proteins recognize 5' PAM sequences of TTTN or CTTN. The skilled person appreciates that by selecting a specific effector protein a certain PAM sequence may be recognized.

The target sequence may be any DNA sequence or RNA sequence. The target sequence may be a sequence within RNA selected from the group consisting of messenger RNA (mRNA), pre-mRNA, ribosomal RNA (rRNA), transfer RNA (tRNA), micro-RNA (miRNA), small interfering RNA (siRNA), small nuclear RNA (snRNA), small nucleolar RNA (snoRNA), double stranded RNA (dsRNA), non-coding RNA (ncRNA), long non-coding RNA (IncRNA), and small cytoplasmic RNA (scRNA). In particular, in the case of Cpf1 nuclease, the target sequence may be any DNA sequence, for example, any sequence of single-stranded DNA (ssDNA) or double-stranded DNA (dsDNA).

In particular, the target sequence is any DNA sequence. The target nucleic acids may then comprise, or consist essentially of, DNA. The target DNA can be ssDNA or dsDNA. In case of ssDNA, there may not be any preference or requirement for a PAM sequence in the target DNA. However, when the target DNA is dsDNA, a PAM is usually present adjacent to the target sequence of the target DNA. The source of the target DNA can be any source as described herein, such as obtained from any sample as described herein.

The target DNA can be a viral DNA (*e.g.,* a genomic DNA of a DNA virus). As such, the system, agents, devices, kits and methods as described herein can each be used for detecting the presence of a viral DNA amongst a population of nucleic acids in any sample, as described herein.

Examples of target DNAs include viral DNAs selected from papovavirus, such as human papillomavirus (HPV) and polyoma virus; hepadnavirus, such as Hepatitis B Virus (HBV); herpesvirus, such as herpes simplex virus (HSV), varicella zoster virus (VZV), epstein-barr virus (EBV), cytomegalovirus (CMV), herpes lymphotropic virus, Pityriasis Rosea, and kaposi's sarcoma-associated herpesvirus; adenovirus, such as atadenovirus, aviadenovirus, ichtadenovirus, mastadenovirus, and siadeno virus; poxvirus, such as smallpox, vaccinia virus, cowpox virus, monkeypox virus, orf virus, pseudocowpox, bovine papular stomatitis virus, tanapox virus, yaba monkey tumor virus, molluscum contagiosum virus (MCV); parvovirus, such as adeno-associated virus (AAV), Parvovirus B19, human bocavirus, bufavirus, and human parv4 Gl); Gemini viridae; Nanoviridae; and Phycodnaviridae.

The target DNA may be parasite DNA. The target DNA may be bacterial DNA, *e.g.,* DNA of a pathogenic bacterium, such as DNA of *Bacillus anthracis.* The target DNA may be from any gram-negative bacterium or gram-positive bacterium, such as a *Mycobacterium.* As such, the system, agents, devices, kits and methods as described herein can each be used for detecting the presence of a bacterial DNA amongst a population of nucleic acids in any sample, as described herein.

Examples of target DNAs include bacterial DNAs selected from the bacteria genera *Streptococcus; Staphylococcus; Pseudomonas; Chlamydophila*; *Ehrlichia; Rickettsia; Orientia*; *Yersinia*; *Burkholderia*; *Shigella; Campylobacter; Salmonella; Clostridium*; *Corynebacterium; Treponema; Neisseria*; *Brucella; Mycobacterium; Lactobacillus; Nocardia*; *Listeria; Francisella,* and *Legionella.*

The target nucleic acids as described herein may be obtained from a biological sample or an environmental sample. The biological sample or environmental sample may originate from a subject as described herein. The biological sample may be obtained from blood, plasma, serum, urine, stool, sputum, mucous, lymph fluid, synovial fluid, bile, ascites, pleural effusion, seroma, saliva, cerebrospinal fluid, aqueous or vitreous humor, or any bodily secretion, a transudate, an exudate, such as fluid obtained from an abscess or any other site of infection or inflammation), or fluid obtained from a joint, such as a normal joint or a joint effected by disease, such as rheumatoid arthritis, osteoarthritis, gout or septic arthritis, a swab of skin or mucosal membrane surface, or a combination thereof. Preferably, the biological sample may be obtained from blood, plasma, serum, urine, stool, sputum, mucous, saliva, or any bodily secretion, a transudate, an exudate, such as fluid obtained from an abscess or any other site of infection or inflammation), a swab of skin or mucosal membrane surface, or a combination thereof. The environmental sample may be obtained from food (*e.g.,* fruit, vegetables, meat, beverage, etc.), paper surface, fabric, metal surface, wood or wood surface, plastic surface, soil, water, such as fresh water or waste water, saline water, atmospheric air or other gas sample, or a combination thereof.

The nucleic acid detection system of the invention comprises a polymerase. The polymerase exhibits catalytic activity capable of transferring nucleotide to termini of nucleic acid fragments, such as 3'-hydroxyl termini, to form a polynucleotide tail. The polymerase is an enzyme which is capable of synthesizing nucleic acid chains. The polymerase may comprise DNA polymerase and/or RNA polymerase, depending on the target nucleic acids, CRISPR-Cas system and/or nucleotide. For example, the polymerase may be terminal deoxynucleotidyl transferase (TdT) or Polθ terminal transferase.

In particular, the polymerase as described herein comprises TdT or a derivative thereof. TdT possesses the ability to incorporate nucleotides, in particular thymine, in a template-independent manner using single-stranded DNA or double-stranded DNA as the nucleic acid substrate. Preferably, single-stranded DNA is used as it functions more efficiently as a template.

The nucleic acid detection system of the invention further comprises nucleotides. Nucleotides, or nucleoside phosphates, consist of a nucleoside, which consists of a nucleobase, or nitrogenous base, and ribose, and one or more phosphate groups. As used herein, a nucleotide is a molecule which comprises a nitrogenous base and one or more phosphate groups bound to a ribose or deoxyribose. In particular, the nitrogenous base is pyrimidine or a derivative thereof. The nitrogenous base may be selected from thymine, cytosine, uracil, alloxan, inosine, adenine or derivatives thereof. Preferably, the nucleotides comprise thymine. The nucleotides comprise at least one phosphate group. In particular, the nucleotides comprise 2 or more phosphate groups, such as 3 phosphate groups. Preferably, the nucleotides are nucleoside triphosphates. More preferably, the nucleotides are deoxythymidine triphosphates (dTTP) or derivatives thereof.

The polymerase and the nucleotide are selected as such that the polymerase is capable of polymerizing the nucleotide to form a polynucleotide (tail).

According to the invention as described herein, such polynucleotide tail is formed and/or attached to the cleaved individual fragments of nucleic acid reporter molecules, in particular *via* the 3'-hydroxyl termini of the fragments. As such, a polynucleotide attached to (cleaved) fragments is coined a polynucleotide tail. The polynucleotide tail as described herein may comprise at least 5 nucleotides, as described herein. The polynucleotide tail may comprise 100 nucleotides or more, such as 150 or more, or 200 or more. For example, the polynucleotide tail may comprise 100-500 nucleotides, such as 150-400, or 200-300. The polynucleotide tail may comprise 5 nucleotides or more and 100 nucleotides or less, such as 15-75, 20-70, or 25-65. Typically, the longer the polynucleotide tail, the better the detection of target molecules, *i.e*., a more visible result. The polynucleotide tail may comprise different nucleotides, such as 2 or more different nucleotides, *e.g.,* 3, 4 or 5 different nucleotides. Preferably, the polynucleotide tail has only one type of nucleotide, for example, a thymine polynucleotide tail.

In an embodiment, a nucleic acid detection system as described herein is provided wherein the nucleotide comprises thymine, such that the formed polynucleotide tail is a poly(thymine) tail, or poly-T tail. Such poly-T tails are not found in nature. Poly-T tails advantageously chelate metal, in particular copper. The capacity of poly-T tails to chelate metal is significantly higher than that of poly-A tails, poly-G tails and poly-C tails (Liu, *et al.* 2013).

The polynucleotide tail attached to a terminus of the nucleic acid fragments, such as the 3'-hydroxyl terminus, comprises metal-binding sites capable of binding metal. The skilled person readily understands that the nitrogenous base of the nucleotide comprises one or more metal-binding sites (*i.e.,* nitrogen and/or oxygen atoms present in the nucleotide), for example, the N3 position of thymine and cytosine, and the N7 position of adenine and guanine.

The nucleic acid detection system as described herein may further comprise metal which is capable of chelating to the metal-binding sites of the polynucleotide tails. In particular, when the metal as described herein is chelated to a metal-binding site, the oxidation state is preferably 0. The metal may comprise metal ions and/or metal atoms. Preferably, the polynucleotide tail has metal-binding sites capable of binding metal ions. The metal may be in the form of particles, such as nanoparticles. The metal may be any metal or metallic element, such as a transition metal. In particular, the metal is selected from elements in groups 1, 2, 11, and/or 12 of the periodic table. Preferably, the metal comprises nickel, cobalt, copper, silver and/or gold. More preferably, copper, silver and/or gold, and even more preferably copper because of an advantageous Stoke-shift. The metal may have any possible oxidation state, such as +3, +2 +1 or 0. Preferably, the oxidation state of the metal is +2 or +1. More preferably, the oxidation state of the metal is +1. Measurements with the detection system as described herein may be interfered with by molecules which are present in the sample, such interfering molecules may be metals, reductants, oxidants, RNase, DNase, etc. This interference may be diminished by, for example, lowering the concentration of the interfering molecule.

In an embodiment, a nucleic acid detection system as described herein is provided wherein the polynucleotide tail specifically chelates copper, in particular Cu¹⁺.

After targeting the effector protein to a target sequence of a target molecule, such as a target nucleic acid, *e.g.,* target pathogenic nucleic acid, the target molecule may be cleaved. This initial cleaving may then unleash further cleavage activity of the effector protein. For example, in the case of Cpf1 effector proteins and derivatives thereof, such target-activated cleavage activity (also referred to herein as "target-activated nucleic acid cleavage activity") may then specifically be used to cleave nucleic acid reporter molecules, such as single-stranded nucleic acid, *e.g.,* single-stranded DNA, thereby forming, or generating, nucleic acid reporter fragments (or, fragments).

In particular, the effector protein in the detection system as described herein exhibits target-activated nucleic acid cleavage activity capable of generating nucleic acid fragments by cleaving nucleic acid reporter molecules, specifically single-stranded nucleic acid strands (especially in case of Cpf1), preferably wherein the reporter molecules are blocked (end-capped) at the 3'-termini. The target-activated nucleic acid cleavage activity of the effector protein as described herein may be specific or non-specific, DNase and/or RNase cleavage activity. Preferably, the target-activated cleavage activity is target-activated single-stranded DNase or double-stranded DNase cleavage activity, more preferably target-activated ssDNase cleavage activity.

Preferably, the nucleic acid reporter molecules are blocked at the 3'-termini (*i.e.,* 3'-hydroxyl termini). The 3'-blocked nucleic acid reporter molecules have a 3'-terminus which is blocked by, for example, circularization, such as using chemical or enzymatic intramolecular ligation, or end-capping the 3'-terminus with, *e.g.,* phosphate, dideoxynucleotide (ddNTP), inverted dNTP, C3 spacer, or amino. It is important that the nucleic acid reporter molecules have blocked 3'-termini to prevent unwanted formation of polynucleotide tails, for example, in the case where the CRISPR-Cas system (*i.e.,* effector protein) is not yet activated. Target-activated effector protein cleaves nucleic acid reporter molecules, thereby forming nucleic acid fragments having 3'-hydroxyl termini which may then suitable act as templates to form polynucleotide tails. As the nucleotides have metal-binding sites, the addition of metal thereto results in metal (nano)clusters. Certain metals, such as Cu⁰, may undergo a visible red color shift when the metal clusters are exposed to ultraviolet radiation.

As is apparent to the skilled person the nucleic acid reporter molecules as described herein should preferably not contain the target sequence as it would lead to unwanted activation of the CRISPR-Cas system. Neither should the reporter molecules contain stretches of repetitive nucleotides corresponding to the same type of nucleotide in the polynucleotide tail or form a base pair with the nucleotide in the tail. For example, when poly-T tails are formed, the reporter molecules preferably do not have stretches of repetitive nucleotides comprising thymine and/or adenine.

Accordingly, with the invention as described herein metal clusters can be formed which, upon exposure to electromagnetic radiation of certain wavelength(s), provide a signal to the observer. In particular, such a signal is a visible color shift.

The nucleic acid detection system as described herein may thus comprise the formation of metal clusters, wherein a metal cluster comprises a fragment to which is attached a polynucleotide tail having metal-binding sites which are capable of chelating metal. Preferably, the polynucleotide tail is attached to a 3'-hydroxyl terminus of the fragment. In particular, the metal is selected such that upon exposure to electromagnetic radiation, *e.g.,* ultraviolet radiation, a (visible) color shift may be detected. Preferably, the ultraviolet radiation has a wavelength of 300-400 nm. These metal (nano)clusters, such as copper clusters, surprisingly exhibit great potential for fluorescent bioassays due to the advantages of high fluorescence yield, good photo-stability and a visible emission (orange/red) when excited with UV-light. Fluorescence spectra of, for example, copper clusters, have an emission peak at about 615 nm when excited at 340 nm, which makes copper clusters well suited for detection in complex biological matrices as this significant Stokes-shift enables removal of strong background signals.

In a preferred embodiment, a nucleic acid detection system is provided comprising a Cas12a effector protein, a Cas12a guide RNA having a guide sequence, the guide sequence being capable of targeting the effector protein to a target sequence of a target, and TdT polymerase. Preferably the system further comprises thymine, a group 2 metal, preferably copper, and 3'-terminus blocked (end-capped) nucleic acid reporter molecules.

There is provided herein a nucleic acid detection system as described herein for use in medical and/or detection applications. The detection system may be for use as a medicament, or as a medical device. Preferably, the nucleic acid detection system for use in medical applications is a nucleic acid fluorescence detection system.

In an embodiment, the nucleic acid detection system as described herein is used in detection applications, for example, to identify anthrax bacterium in environmental samples, such as anthrax (hoax) letters (in Dutch: "poederbrieven").

The term "medical applications" as used herein is meant to include, for example, methods for diagnosing a disease state of a subject. The term "subject" as used herein is meant to include the human and animal body and plants, and the terms "individual" and "patient". The terms "human" and "nonhuman" as used herein, are meant to include all animals, such as mammals, including humans. The term "individual" as used herein is meant to include any human or nonhuman entity. Humans and/or non-humans, such as domesticized animals (*i.e.,* pets, livestock, zoo animals, equines, etc.), may be subjected to the medical applications.

The invention also provides a use of a nucleic acid detection system as described herein as a diagnostic agent. The diagnostic agent may be used to diagnose a disease state of a subject as described herein.

There is also provided herein a diagnostic agent comprising the nucleic acid detection system as described herein.

There is further provided a nucleic acid detection system as described herein for use in detecting *in vitro, in vivo or ex vivo* pathogenic nucleic acids, wherein preferably the pathogenic nucleic acids is pathogenic DNA. Preferably, the nucleic acid detection system is a nucleic acid fluorescence detection system.

The nucleic acid detection system as described herein can be embodied on devices, in particular diagnostic devices. Hence, the invention further provides a diagnostic device, comprising a nucleic acid detection system as described herein. The diagnostic device optionally comprises a source of electromagnetic radiation, in particular a source of ultraviolet radiation.

The device may be capable of defining multiple individual discrete volumes within the device, or a single individual discrete volume. As used herein an "individual discrete volume" refers to a discrete space, such as a container, receptacle, or other defined volume or space that can be defined by properties that prevent and/or inhibit migration of target molecules, for example a volume or space defined by physical properties such as walls a well or tube, which may be impermeable or semipermeable, or as defined by other means such as chemical, diffusion rate limited, electro-magnetic, or light illumination, or any combination thereof that can contain a sample within a defined space. The individual discrete volume may typically include a fluid medium (*e.g.,* an aqueous solution, an oil, a buffer, etc.). Exemplary discrete volumes or spaces useful in the disclosed methods include tubes (*e.g.,* centrifuge tubes, micro-centrifuge tubes, test tubes, cuvettes, and conical tubes), bottles (*e.g.,* glass bottles, plastic bottles, ceramic bottles, Erlenmeyer flasks, and scintillation vials), wells (such as wells in a plate), plates, pipettes, and pipette tips.

The CRISPR effector protein may be bound to each discrete volume in the device. Each discrete volume may comprise a different guide RNA specific for a different target molecule. Accordingly, samples comprising target molecules may be exposed to one or more of the discrete volumes each comprising a guide RNA specific for a target molecule. Each guide RNA may preferably capture a specific target molecule from the sample, such that the sample does not need to be divided into separate assays.

A dosimeter or badge may be provided with the device as described herein that serves as a sensor or indicator, such that the wearer may be notified of exposure to certain microbes or other agents. Providing such a dosimeter or badge with the device may be particularly useful for first responders, surveillance of soldiers or other military personnel, as well as clinicians, researchers, and hospital staff, in order to provide information relating to exposure to potentially dangerous agents as quickly as possible, for example for biological or chemical warfare agent detection. Such a surveillance badge may be used for preventing exposure to dangerous microbes (or pathogens) in, for example, immunocompromised patients, burn patients, patients undergoing chemotherapy, children, or elderly.

Near-real-time microbial diagnostics may be beneficial for food, clinical, industrial, and other environmental settings. Hence, the present invention may be used for rapid detection of, for example, foodborne pathogens, using one or more guide RNAs that are specific to one or more target pathogens.

The invention further provides a kit-of-parts for detecting nucleic acids, comprising:
i) a first container (A) which comprises:
   a CRISPR-Cas system, preferably as described herein, and preferably further comprising nucleic acid reporter molecules, preferably as described herein, preferably single-stranded nucleic acids;
ii) a second container (B) which comprises:
   polymerase and nucleotides, preferably as described herein, and
iii) a third container (C) which comprises:
   metal, preferably as described herein, preferably together with a reductant. The kit-of-parts optionally comprises a fourth container (D) which comprises a source of electromagnetic radiation. The source of electromagnetic radiation may in particular be a source of ultraviolet radiation. The first, second, and/or third container may further comprise a buffer, for example, containing acetate, having a pH of 7-9, such as about 7.5-8.5. The nucleic acid report molecules are preferably blocked at the 3'-termini to prevent elongation of the polymerase of the second container.

There is also provided herein a kit-of-parts for detecting nucleic acids, comprising:
i) a first container (A) which comprises:
   a CRISPR-Cas system, preferably as described herein, and preferably further comprising nucleic acid reporter molecules, preferably as described herein, preferably single-stranded nucleic acids;
ii) a second container (B) which comprises:
   polymerase, preferably as described herein;
iii) a third container (C) which comprises:
   nucleotides, preferably as described herein, and
iv) a fourth container (D) which comprises:
   metal, preferably as described herein, preferably together with a reductant.
The kit-of-parts may optionally comprise a fifth container (E) which comprises a source of electromagnetic radiation. The source of electromagnetic radiation may in particular be a source of ultraviolet radiation. The first, second, third and/or fourth container may further comprise a buffer, for example, containing acetate, having a pH of 7-9, such as about 7.5-8.5. The nucleic acid report molecules are preferably blocked at the 3'-termini to prevent elongation of the polymerase of the second container.

There is further provided herein a kit-of-parts for detecting nucleic acids, comprising:
i) a first container (A) which comprises:
   a CRISPR-Cas system, preferably as described herein, and preferably further comprising nucleic acid reporter molecules, preferably as described herein, preferably single-stranded nucleic acids, and
ii) a second container (B) which comprises:
   polymerase, nucleotides, and metal, each preferably as described herein, preferably together with a reductant.
The kit-of-parts may optionally comprise a third container (C) which comprises a source of electromagnetic radiation. The source of electromagnetic radiation may in particular be a source of ultraviolet radiation. The first and/or second container may further comprise a buffer, for example, containing acetate, having a pH of 7-9, such as about 7.5-8.5. The nucleic acid report molecules are preferably blocked at the 3'-termini to prevent elongation of the polymerase of the second container.

With the kits-of-parts as described herein the preferably present reductant, which is in particular a reductant as described herein, ensures an oxidation state of the metal which is suitable for chelation with the nucleotides, thereby forming metal clusters, as described herein. The reductant may, for example, be a salt comprising ascorbate, such as sodium ascorbate.

The invention further provides a method for detecting nucleic acids, preferably pathogenic DNA, comprising:
i) target-activating the nucleic acid cleavage activity of a CRISPR-Cas system as described herein and allowing the activated CRISPR-Cas system to generate nucleic acid fragments by cleaving nucleic acid reporter molecules, preferably as described herein;
ii) adding polymerase and nucleotides to at least part of the nucleic acid fragments to form a polynucleotide tail attached to the nucleic acid fragments, wherein preferably the polymerase and/or nucleotides are as described herein;
iii) adding metal, preferably as described herein, preferably together with a reductant, to bind the metal to the polynucleotide tail thereby forming metal clusters, and
iv) exposing the metal clusters to electromagnetic radiation, preferably ultraviolet radiation, in particular having a wavelength of 300-400 nm.

Target-activation is achieved by targeting the effector protein of the CRISPR-Cas system to a target sequence of a target, wherein the target may be a target nucleic acid as described herein. Preferably, the target is a pathogenic nucleic acid. Target-activation may comprise providing the CRISPR-Cas system to a sample, such as any sample described herein. If the sample contains the target sequence, then the CRISPR-Cas system will be activated and will (subsequently) cleave the nucleic acid reporter molecules.

In particular, the nucleic acid reporter molecule in the method is as described herein. The nucleic acid reporter molecule is preferably blocked at the 3'-terminus to prevent, for example, elongation of the polymerase in the absence of nuclease activity. Preferably, when the CRISPR-Cas system comprises Cpf1 as the effector protein, the nucleic acid reporter molecule is ssDNA, in particular ssDNA having their 3'-termini blocked. The low cost and adaptability of the detection system as described herein lends itself to a number of applications. The generated fragments preferably have 3'-hydroxyl ends such that polynucleotide tails are attached at said ends.

The method may further comprise a step of inactivating the CRISPR-Cas system. The CRISPR-Cas system may be inactivated by any method known from the art, *e.g.,* chemical inactivation, radiative inactivation, etc. Preferably, the method comprises a step of denaturing the effector protein. Preferably, the inactivating step may be, for example, a heating step to deactivate the CRISPR-Cas system such that the effector protein is denatured. As a possible result thereof, unwanted side-reactions are circumvented, such as cleavage of the polynucleotide tail formed at step ii) by the effector protein. The temperature and duration of the optional heating step depend on the effector protein. The CRISPR-Cas system may be heated at a temperature above room temperature, such as 30 °C or more. In particular, the temperature of the heating step is 35 °C or more and 90 °C or less, such as 40-80 °C, 45-75 °C, or 50-70 °C. The CRISPR-Cas system may be heated for at least 30 seconds, such as 1 min or longer. In particular, the CRISPR-Cas system is heated for 1-20 min, such as 3 min or longer, 5 or longer, 7 min or longer, or 10 or longer, and 17 min or shorter, 15 min or shorter, or 12 min or shorter. Preferably, the duration of the heating step is approximately 10-20 min. The inactivation step may preferably be performed after step i), such as between steps i) and ii).

With step i) of the method the temperature at which the target-activation and/or generation of fragments is performed depends on the effector protein. For example, the step may be performed at room temperature. In particular, the step may be performed at a temperature of at least 20 °C, such as 25-60 °C. Preferably, step i) is performed at a temperature of 30-55 °C to assist the formation of nucleic acid reporter fragments. The CRISPR-Cas system may be target-activated during at least 1 min. In particular, the duration of step i) may be 2 min or more, preferably at least 15 minutes. In some embodiments, the duration of step i) may be 90 min or less, such as 60 min or less, 40 min or less, 35 min or less, 30 min or less, 25 min or less, 20 min or less, or 15 min or less, for example, 3-50 min, or 4-45 min,. Preferably, the duration of step i) is approximately 5-40 min, such as 6-35 min or 7-30 min. Shortening the duration of 60 min of step i) allows for faster total detection, yet results in a decreased signal. In a preferred embodiment, the duration of step i) is between 40-70 minutes.

Step i) of the method as described herein may be performed in weak basic conditions, *i.e*., at a pH of 7.5 or higher. In particular, the pH is 7.6 or higher and 9 or lower. Preferably, the pH is about 7.7-8.5. A buffer may be present at any step of the method as described herein. For example, the buffer may contain acetate, having a pH of 7-9, such as about 7.5-8.5. The buffer may comprise a cation, such as divalent metal ions, *e.g.,* Mg²⁺, as it may be required by the polymerase and/or effector protein. Other divalent metal ions that may be present in the buffer are Co²⁺, Mn²⁺ and/or Zn²⁺. In an exemplary embodiment using Cas12a as the effector protein and TdT as the polymerase, an acetate containing buffer is used.

A reductant, for example, a salt, may be added during the method, for example, at step iii) to provide a reducing agent, or reductant, to reduce any metal ion present. By reducing the metal (ions), metal ions, such as Cu¹⁺, may be formed. These metal atoms subsequently act as scaffold nucleation sites for the formation of the metal nanoclusters, wherein preferably the metal has oxidation state 0. In particular, the salt provides a weak and/or slow reductant such that metal ions have time to chelate to the polynucleotide tails. Suitable salts comprise ascorbate, such as sodium ascorbate.

The method as described herein may comprise distributing a sample or set of samples into one or more individual discrete volumes comprising a CRISPR-Cas system as described herein, and optionally nucleic acid reporter molecules. The sample or set of samples may be incubated under conditions sufficient to allow the guide sequence of the gRNA to target the effector protein to a target sequence of a target molecule. The then target-activated CRISPR-Cas effector protein exhibits nucleic acid cleavage activity, as described herein, capable of cleaving the nucleic acid reporter molecules, thereby generating fragments that act as polynucleotide templates as described herein. The further addition of metal may result in the formation of metal clusters which, upon exposure to, for example, ultraviolet radiation, may emit a detectable color shift indicating the presence of a certain target molecule.

The target nucleic acids may be diagnostic for a disease state. In particular, the disease state is selected from infectious diseases, organ diseases, blood diseases, immune system diseases, cancers, brain and nervous system diseases, endocrine diseases, pregnancy or childbirth-related diseases, inherited diseases, environmentally-acquired diseases, or a combination thereof, preferably infectious diseases.

The method as described herein may be directed to detecting the presence of one or more microbial agents in a sample, such as any sample described herein, for example, obtained from a subject as described herein.

The microbe may be a bacterium (including spores), fungus, yeast, protozoa, parasite, or virus, preferably a bacterium. Accordingly, the method disclosed herein can be adapted for use in other methods (or in combination) with other methods that require quick identification of microbe species, monitoring the presence of microbial proteins (antigens), antibodies, antibody genes, detection of certain phenotypes (*e.g.,* bacterial resistance), monitoring of disease progression and/or outbreak, and antibiotic screening.

The method may further comprise a step of observing a shift in color upon exposing the metal clusters to electromagnetic radiation. In particular, the observation may be made by eye, *i.e*., preferably without requiring additional optical instruments.

The invention further provides a method for detecting and/or monitoring nucleic acids in a subject, particularly *ex vivo* or in *vitro,* wherein preferably the nucleic acids are microbial nucleic acids, more preferably pathogenic nucleic acids. The method comprises the steps of the method for detecting (target) nucleic acids as described herein. With this method, a sample, such as a biological sample as described herein, is taken from a subject for detecting and/or monitoring nucleic acids that are present in the sample. Preferably, microbial nucleic acids, and more preferably pathogenic nucleic acids, are detected and/or monitored. Even more preferably, the method is used for detecting and/or monitoring (the presence of) pathogenic DNA, such as viral and/or bacterial DNA, in a subject.

The invention further provides a method for diagnosing a disease state of a subject, particularly *ex vivo* or *in vitro.* The method comprises the steps of the method for detecting nucleic acids as described herein. The disease state is preferably selected from the disease states as described herein.

The invention has been described by reference to various embodiments, and methods. The skilled person understands that features of various embodiments and methods can be combined with each other.

All references cited herein are hereby completely incorporated by reference to the same extent as if each reference were individually and specifically indicated to be incorporated by reference and were set forth in its entirety herein.

The use of the terms "a" and "an" and "the" and similar referents in the context of describing the invention (especially in the context of the claims) are to be construed to cover both the singular and the plural, unless otherwise indicated herein or clearly contradicted by context. The terms "comprising", "having", "including" and "containing" are to be construed as open-ended terms (*i.e.,* meaning "including, but not limited to") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. The use of any and all examples, or exemplary language (*e.g.,* "such as") provided herein, is intended merely to better illuminate the invention and does not pose a limitation on the scope of the invention unless otherwise claimed. No language in the specification should be construed as indicating any non-claimed element as essential to the practice of the invention. For the purpose of the description and of the appended claims, except where otherwise indicated, all numbers expressing amounts, quantities, percentages, and so forth, are to be understood as being modified in all instances by the term "about". Also, all ranges include any combination of the maximum and minimum points disclosed and include any intermediate ranges therein, which may or may not be specifically enumerated herein.

When referring to a noun (*e.g.,* an effector protein) in the singular, the plural is meant to be included, or it follows from the context that it should refer to the singular only.

Preferred embodiments of this invention are described herein. Variation of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject-matter recited in the claims appended hereto as permitted by applicable law. Moreover, any combination of the above-described elements in all possible variations thereof is encompassed by the invention unless otherwise indicated herein or otherwise clearly contradicted by context. The claims are to be construed to include alternative embodiments to the extent permitted by the prior art.

For the purpose of clarity and a concise description features are described herein as part of the same or separate embodiments, however, it will be appreciated that the scope of the invention may include embodiments having combinations of all or some of the features described.

Hereinafter, the invention will be illustrated in more detail, according to specific examples. However, the invention may be embodied in many different forms and should not be construed as being limited to the embodiments set forth herein. Rather, these example embodiments are provided so that this description will be thorough and complete, and will fully convey the scope of the invention to those skilled in the art.

### Examples

### Materials

All chemicals are commercially available and were used as obtained. Sodium L-ascorbate (#A7631-25G) was obtained from Sigma-Aldrich. Copper(II) sulfate pentahydrate (#203165-50G) was obtained from Sigma-Aldrich. LbCas12a (#M0653T) was obtained from Engen. Terminal Deoxynucleotidyl Transferase (#M0315L) was obtained from New England Biolabs together with matching CoCl₂ (2.5 mM stock), TdT buffer (50 mM Potassium Acetate, 20 mM Tris-acetate, 10 mM Magnesium Acetate; pH 7.9 at 25 °C), and dTTP (ThermoFisher #R0171/New England Biolabs # N0446S). A HTX Synergy multi-mode plate reader (apparatus) was used from Biotek instruments, Inc. A plasmid encoding partial gene of anthrax toxin lethal factor amino acids 253-380, GCTTTTGCATATTATATCGAGCCACAGC ATCGTGATGTTTTACAGCTTTATGCACCGGAAGCTTTTAATTACATGGATAAAT TTAACGAACAAGAAATAAATCTATCCTTGGAAGAACTTAAAGATCAACGGATG CTGGCAAGATATGAAAAATGGGAAAAGATAAAACAGCACTATCAACACTGGAG CGATTCTTTATCTGAAGAAGGAAGAGGACTTTTAAAAAAGCTGCAGATTCCTA TTGAGCCAAAGAAAGATGACATAATTCATTCTTTATCTCAAGAAGAAAAAGAG CTTCTAAAAAGAATACAAATTGATAGTAGTGATTTTTTATCTACTGAGGAAAAA GAGTTTTTAAAAAAGCTACAAATTGATATTCGTGATTC, was synthesized and cloned into pTwist Amp High Copy plasmid by Twist Biosciences (San Francisco). Reporter ssDNA oligonucleotides according to (Luo *et al*., 2017) were ordered at Isogen Life Sciences (Utrecht, The Netherlands): 5'-AAC TAT GCA ACC TAC TAC CTC T-OH-3' (RepU) and 5'-AAC TAT GCA ACC TAC TAC CTC T-PO₃-3' (RepB). Designed crRNAs for recognition of the anthrax toxin lethal factor were ordered at Isogen Life Sciences with the following ssRNA sequences: 5'-UAA UUU CUA CUA AGU GUA GAU CAU AUU AUA UCG AGC CAC AGC-OH-3' (crRNA1); 5'-UAA UUU CUA CUA AGU GUA GAU UGC ACC GGA AGC UUU UAA UUA C-OH-3' (crRNA2); and 5'-UAA UUU CUA CUA AGU GUA GAU GCU CAA UAG GAA UCU GCA GC-OH-3' (crRNA3).

### Methods

### In vitro Cas12 activation

Presence of the gene sequence was evaluated *in vitro* by addition of the plasmid carrying the partial anthrax lethal factor gene to a reaction vessel. The final solution contained: 50 nM LbCas12a, 62.5 nM crRNA2 (unless results are labeled with crRNA1' or 'crRNA3'), 100 nM reporter-3'PO₃ (RepB), 2 nM plasmid (unless otherwise stated) and 1x TdT buffer (New England Biolabs). Total used reaction volume was 50 µl. The samples were incubated at 37 °C for 60 min, after which heat inactivation of the LbCas12a was performed by incubation at 70 °C for 15 min.

### Elongation of 3'-hydroxyl fragments into poly-T tails by TdT

Elongation of the 3'-hydroxyl ends was started by addition of terminal deoxynucleotidyl transferase to the products generated in the Cas12a activation step. The added volume of 50 µl contained: 1x TdT buffer (New England Biolabs), 0.5 mM CoCl₂ (New England Biolabs, provided with TdT), 0.8 U/µl TdT, and 8 mM dTTP. The final concentrations in the total final 100 µl volume of the relevant components are therefore: 1x TdT buffer, 0.25 mM CoCl₂, 0.4 U/µl TdT, 4 mM dTTP. The samples were subsequently incubated at 37 °C for 3 hours.

### Synthesis of fluorescent copper nanoclusters

Copper nanocluster synthesis was started by addition of 4 mM ascorbate and 200 µM CuSO₄ and immediate mixing. Samples were transferred to a black 96-well plate without clear bottom. Fluorescence measurements were performed at RT in the HTX synergy plate reader on fluorescence mode with filters: excitation 360/40, emission 590/35. Samples were transferred to Eppendorf tubes or a clear 96-well plate and placed on a UV light source for visual assessment.

### Statistics

Error bars in the figures were calculated from duplicates, calculating the average and standard deviation using the functions 'AVERAGE' and 'STDEV.P' in excel. Background subtracted graphs show the absolute fluorescence value minus the value of an empty well.

### Example 1

### Feasibility study

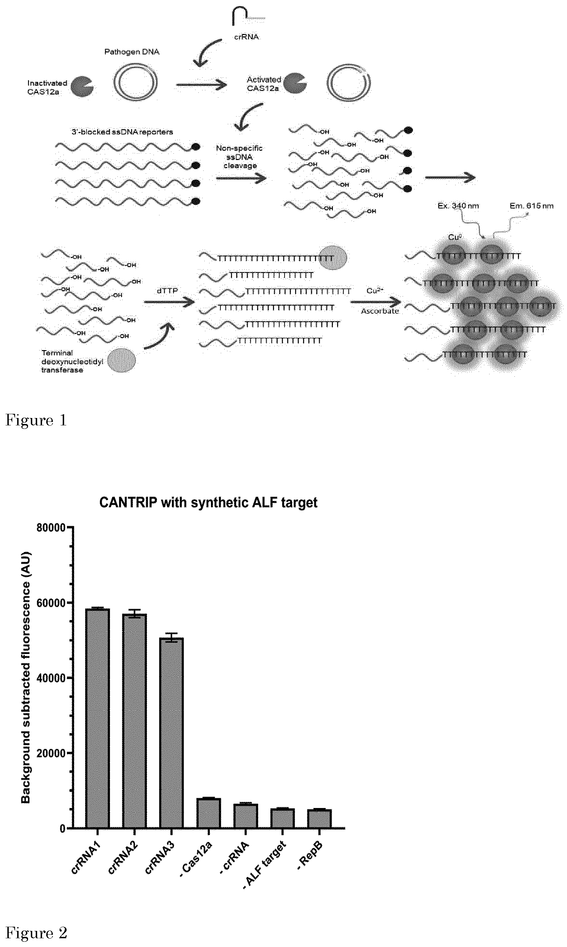

A strategy for specific Cas12a-dependent poly(thymine) formation is given in figure 1. The strategy is divided in essentially three steps. To achieve Cas12a trans-cleavage activation, *Lachnospiraceae bacterium* Cas12a (NEB), a plasmid containing a gene encoding part of Anthrax Lethal Factor (*ALF*; aa 253-380) and the three corresponding crRNAs were employed (see *Materials*). In order to combine the three-step process in a one tube system, a single reaction buffer needed to be established. All three key components, *i.e*., Cas12a, TdT and copper nanoclusters function optimally in weakly basic conditions. TdT is inhibited by high concentrations of chloride ions present in recommended Cas12a buffers, therefore the reaction was performed in an acetate containing buffer (potassium-acetate, tris-acetate and magnesium acetate pH 7.9, see *Materials*). As illustrated in figure 2 a very strong fluorescent signal is emitted in the presence of the complete reaction mix. Each of the three crRNAs caused Cas12a activation and a strong fluorescent signal. Leaving out any of the components in the reaction, crRNA, *ALF* gene, Cas12a enzyme or the blocked reporter, did not result in an observable signal. Thus, both enzymes Cas12a and TdT perform well in these buffer conditions. The presence of 0.25 mM CoCl₂, which is a necessary cofactor for TdT added in step 2, is compatible with downstream copper nanocluster formation.

### Sensitivity study

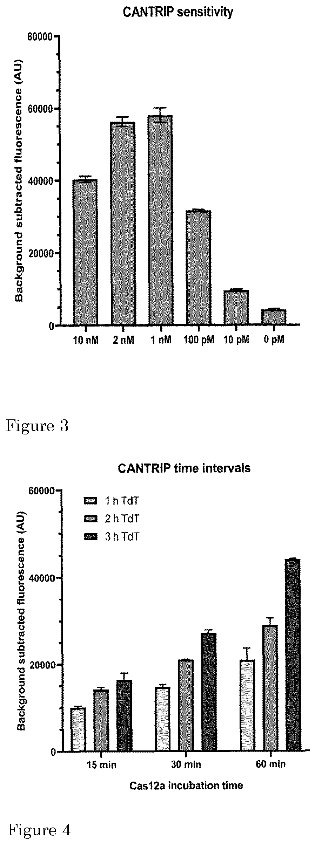

Having established that it is possible to perform the three-step process in a single tube, the sensitivity of the detection method was tested using serially diluted plasmid DNA (figure 3). Based on both the fluorescence measurement and visual assessment, the detection limit of the system as described herein was evaluated at 10 picomolar. To investigate the reaction time, identical reaction mixtures were incubated over varying time intervals. Cas12a was added to each of the tubes and left for time intervals of 15, 30 and 60 minutes. Subsequently TdT was added and incubated for time intervals of 1, 2 and 3 hours. The result, given in figure 4, shows that for both enzymes maximal incubation times (1 hr for Cas12a, and 3 hr for TdT) promote the signal intensity. The shortest times allowing detection above background using the fluorescence plate reader is 15 min for Cas12a and 1 hr for TdT, but the signal is hard to discriminate by eye.

## Claims

1. A nucleic acid detection system, comprising: a CRISPR-Cas system which comprises an effector protein and one or more guide RNAs having a guide sequence, the guide sequence being capable of targeting the effector protein to a target sequence of a target, and the effector protein exhibiting target-activated nucleic acid cleavage activity capable of cleaving nucleic acid reporter molecules to generate nucleic acid fragments; and a polymerase exhibiting catalytic activity capable of transferring nucleotides to the fragments to form polynucleotide tails having metal-binding sites capable of chelating metal, wherein preferably the detection system is a nucleic acid fluorescence detection system.

2. The nucleic acid detection system according to claim 1, wherein the CRISPR-Cas system is a class 2 CRISPR-Cas system, preferably comprising effector protein Cas12 or an effector protein having similar cleavage activity as Cas12, more preferably wherein the effector protein is Cas12a (Cpf1).

3. The nucleic acid detection system according to claim 1 or 2, wherein the polymerase comprises DNA polymerase and/or RNA polymerase, preferably terminal deoxynucleotidyl transferase.

4. The nucleic acid detection system according to any one of claims 1-3, further comprising nucleotides, wherein the nucleotides preferably comprise thymine, cytosine, uracil, alloxan, inosine, adenine and/or one or more derivatives thereof, more preferably deoxythymidine triphosphate.

5. The nucleic acid detection system according to any one of claims 1-4, further comprising metal, wherein the metal is particularly selected from elements in groups 1, 2, 11, and/or 12 of the periodic table, preferably group 2, more preferably nickel, cobalt, copper, silver and/or gold, even more preferably copper.

6. The nucleic acid detection system according to any one of claims 1-5, wherein the target comprises nucleic acids, in particular RNA and/or DNA, preferably single-stranded DNA or double-stranded DNA, more preferably double-stranded DNA, and preferably wherein the nucleic acids are microbial nucleic acids, more preferably pathogenic nucleic acids.

7. The nucleic acid detection system according to any one of claims 1-6, further comprising nucleic acid reporter molecules, preferably single-stranded nucleic acids, preferably wherein the nucleic acid reporter molecules are blocked at a 3'-terminus.

8. Use of a nucleic acid detection system according to any one of claims 1-7 as a diagnostic agent.

9. A diagnostic device, comprising one or more nucleic acid detection systems according to any one of claims 1-8, and optionally comprising a source of electromagnetic radiation.

10. A kit-of-parts for detecting nucleic acids, comprising:
i) a first container (A) which comprises:
a CRISPR-Cas system, preferably as defined in any one of claims 1, 2 or 6, and preferably further comprising nucleic acid reporter molecules, preferably as defined in claim 7, preferably single-stranded nucleic acids;
ii) a second container (B) which comprises:
polymerase, preferably as defined in claim 1 or 3, and nucleotides, preferably as defined in claim 1 or 4;
iii) a third container (C) which comprises:
metal, preferably as defined in claim 1 or 5, preferably together with a reductant, and
iv) optionally a fourth container (D) which comprises:
a source of electromagnetic radiation.

11. A method for detecting nucleic acids, preferably pathogenic DNA, comprising:
i) target-activating the nucleic acid cleavage activity of a CRISPR-Cas system as defined in any one of claims 1, 2 or 6 and allowing the activated CRISPR-Cas system to generate nucleic acid fragments by cleaving nucleic acid reporter molecules, preferably as defined in claim 7;
ii) adding polymerase and nucleotides to at least part of the nucleic acid fragments to form a polynucleotide tail attached to the nucleic acid fragments, wherein preferably the polymerase and/or nucleotides are as defined in any one of claims 1, 3 or 4;
iii) adding metal, preferably as defined in claim 1 or 5, preferably together with a reductant, to bind the metal to the polynucleotide tail thereby forming metal clusters, and
iv) exposing the metal clusters to electromagnetic radiation, preferably ultraviolet radiation, in particular having a wavelength of 300-400 nm.

12. The method according to claim 11, wherein the method is a method for detecting and/or monitoring nucleic acids in a subject, preferably *ex vivo* or *in vitro,* wherein preferably the nucleic acids are microbial nucleic acids, more preferably pathogenic nucleic acids.

13. The method according to claim 11, wherein the method is a method for diagnosing a disease state of a subject, preferably *ex vivo* or *in vitro.*

14. The method according to any one of claims 11-13, wherein the nucleic acids are obtained from a biological sample, preferably a biological sample obtained from blood, plasma, serum, urine, stool, sputum, mucous, saliva, or any bodily secretion, a transudate, an exudate, such as fluid obtained from an abscess or any other site of infection or inflammation), a swab of skin or mucosal membrane surface, or a combination thereof; or
the target nucleic acids are obtained from an environmental sample, preferably an environmental sample obtained from food, paper surface, fabric, metal surface, wood or wood surface, plastic surface, soil, water, such as fresh water or waste water, saline water, or a combination thereof.

15. The method according to any one of claims 11-14, wherein the nucleic acids are diagnostic for a disease state, preferably a disease state selected from infectious diseases, organ diseases, blood diseases, immune system diseases, cancers, brain and nervous system diseases, endocrine diseases, pregnancy or childbirth-related diseases, inherited diseases, environmentally-acquired diseases, or a combination thereof.
